(19) Europäisches Patentamt
European Patent Office
Office européen des brevets

(11) **EP 3 578 991 B1**

(12) **EUROPÄISCHE PATENTSCHRIFT**

(45) Veröffentlichungstag und Bekanntmachung des Hinweises auf die Patenterteilung:
**23.12.2020 Patentblatt 2020/52**

(51) Int Cl.:
*G01N 33/84* *(2006.01)*    *G01N 31/22* *(2006.01)*

(21) Anmeldenummer: **19178035.2**

(22) Anmeldetag: **04.06.2019**

(54) **TEST ZUR BESTIMMUNG DER PHOSPHAT-KONZENTRATION**

TEST FOR DETERMINING PHOSPHATE CONCENTRATION

ESSAI DE DÉTERMINATION DE LA CONCENTRATION EN PHOSPHATE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **07.06.2018 DE 102018209082**

(43) Veröffentlichungstag der Anmeldung:
**11.12.2019 Patentblatt 2019/50**

(73) Patentinhaber: **AXAGARIUS GmbH & Co. KG**
**52355 Düren (DE)**

(72) Erfinder:
- **Husmann, Ralph**
**52355 Düren (DE)**
- **Mertens, Lucas**
**52249 Eschweiler (DE)**

(74) Vertreter: **dompatent von Kreisler Selting Werner - Partnerschaft von Patent- und Rechtsanwälten mbB**
**Deichmannhaus am Dom**
**Bahnhofsvorplatz 1**
**50667 Köln (DE)**

(56) Entgegenhaltungen:
**WO-A1-2017/044806    US-A- 5 858 797**

- **BARTELS P C ET AL: "A kinetic study on the influence of the parameters in the determination of inorganic phosphate by the molybdenum blue reaction", CLINICA CHIMICA ACTA, ELSEVIER BV, AMSTERDAM, NL, Bd. 61, Nr. 2, 2. Juni 1975 (1975-06-02), Seiten 135-144, XP025199707, ISSN: 0009-8981, DOI: 10.1016/0009-8981(75)90307-1 [gefunden am 1975-06-02]**

EP 3 578 991 B1

**Beschreibung**

Die vorliegende Erfindung betrifft eine Vorrichtung zur Bestimmung der Konzentration von anorganischem Phosphat, und hierbei insbesondere Orthophosphat, wobei die Vorrichtung eine Testmatrix enthält, die mindestens ein Molybdatsalz, mindestens eine feststoffliche Säure, ein PVP-Derivat mit einem K-Wert von 40 und eine Kombination von zwei chromogenen Reduktionsmitteln aufweist. Die Erfindung betrifft zudem ein Verfahren zur Bestimmung der Orthophosphat-Konzentration in einer flüssigen Probe unter Verwendung der erfindungsgemäßen Vorrichtung und ein Verfahren zur Sensitivitätserhöhung einer Testvorrichtung.

Die Kombinationen von zwei chromogenen Reduktionsmitteln, die in der Erfindung verwendet werden, sind in den Ansprüchen präzisiert worden.

Phosphor wird in zahlreichen industriellen Produkten verwendet. Er gelangt zum Beispiel durch Düngemittel und Waschmittel in die Umwelt und liegt meist in Form von Orthophosphat oder Polyphosphat vor. Die Bedeutung des Pflanzennährstoffs Phosphor ist hier durch sein Eutrophierungspotenzial begründet. Weiterhin ist die analytische Bestimmung von Orthophosphat neben der Umweltanalytik auch in anderen Gebieten von großer Bedeutung wie bspw. in der klinischen und agrokulturellen Analytik.

Im klinischen Bereich ist die Bestimmung von Phosphat in Körperflüssigkeiten wie Urin oder Blut wichtig, da der Nachweis erhöhter Phosphatkonzentrationen in diesen Flüssigkeiten die Diagnose von Krankheiten wie Urämie oder chronischen Nierenerkrankungen erlaubt, also Krankheiten, die mit einer Phosphatretention einhergehen.

Letztendlich ist die Phosphatbestimmung auch im privaten Bereich in der Qualitätsbestimmung von Aquariumwasser notwendig. Die Bestimmung des gelösten anorganischen Phosphats in wässrigen Flüssigkeiten wird im Allgemeinen gemäß der Methode von Murphy und Riley (1962) durchgeführt (Murphy, J. & Riley, J. P., 1962. A modified single solution method for the determination of phosphate in natural waters. Analytica chim. Acta 27: 31-36). Bei diesem Nachweis bildet das in der wässrigen Flüssigkeit vorhandene Orthophosphat im pH-Bereich von 1,5 zusammen mit Molekülen der Hexamolybdänsäure die Molybdatophosphorsäure. Diese Reaktion wird durch Polyvinylpyrrolidon katalytisch beschleunigt. Durch ein Reduktionsmittel (z. B. Ascorbinsäure) als Teil des Mischreagenz wird die Heteropolysäure ein der zwei Äquivalente reduziert und bildet hierbei blaue Farbkomplexe, die als sogenanntes "Molybdänblau" leuchtendblaue Molybdän(VI)-Molybdän(IV)-Mischoxide darstellen. Die Extinktion des Farbkomplexes ist hierbei proportional zur Konzentration des Phosphor-Molybdatkomplexes und damit zur Konzentration des Phosphats in der Probe. Bei der Durchführung als Schnelltest werden die Reaktionskomponenten in einer Trägermatrix vorgehalten und nach Tränken mit der zu bestimmenden Flüssigkeit die Farbänderung der Testmatrix visuell erfasst. Im Gegensatz zu einem automatisierten reinen Flüssigkeitsassay sind die Testmatrix-basierten Methoden aus dem Stand der Technik relativ unempfindlich, insofern sie eine Nachweisgrenze von 3,0 mg/L Orthophosphat aufweisen.

Das US-Patent Nr. 5,858,797 betrifft einen kolorimetrischen Phosphat-Assay und offenbart die Verwendung eines chromogenen Reduktionsmittels "a reducing dyestuff". Durch die Redoxreaktion wird entsprechend nicht nur der Molybdänblau-Komplex erzeugt, sondern auch das in der Leuko-Form vorliegende Reduktionsmittel in seine farbige Oxidationsform überführt, so dass im Ergebnis ein deutlicherer Farbumschlag resultiert. Das hier offenbarte Verfahren benötigt zur Erhöhung der Messempfindlichkeit einen zusätzlichen Inertfarbstoff wie Malachitgrün oder Ponceaus S (Spalte 2, Zeile 33). Für ein reproduzierbares Ergebnis mit einem Testmatrix-basierten Test ist es entsprechend notwendig, dass sich dieser Inertfarbstoff schnell und vollständig auflöst.

Bartels P.C. et al. ("A kinetic study on the influence of the parameters in the determination of inorganic phosphate by the molybdenum blue reaction", CLINICA CHIMICA ACTA, ELSEVIER BV, AMSTERDAM, NL, Bd. 61, Nr. 2, 2. Juni 1975 (1975-06-02), Seiten 135-144, XP025199707, ISSN: 0009-8981, DOI: 10.1016/0009-8981(75)90307-1) belegten mittels kinetischer Daten, dass Polyvinylpyrrolidon an der Nachweisreaktion für Phosphat teilnimmt. Die Autoren offenbarten auch eine Methode zur Bestimmung der Konzentration von anorganischem Phosphat im Blutserum und Urin, welche die Befreiung der Probelösungen von Proteinen nicht nötig macht.

Die WO 2017/044806 betrifft eine Methode zum *in situ* Nachweis von Peroxycarbonsäurekonzentrationen, die für die Desinfektion von Oberflächen geeignet sind. Diese Methode beruht auf einem drei-Farbstoff-System, wobei die Farbstoffe einen inerten Hintergrundfarbstoff und mindestens einen Redoxfarbstoff umfassen.

Ein Nachteil ist die Tatsache, dass bei vielen Schnelltests und insbesondere bei den besonders empfindlichen Schnelltests, Zusatzreagenzien notwendig sind. Dies sind klassischerweise stark azide Mineralsäuren, wie Salpetersäure, die der zu messenden Probe in einem ersten Schritt hinzugegeben werden und erst nach Ausbildung dieser Mischung das Testverfahren durchgeführt werden kann. Damit wird das Messverfahren fehleranfälliger und zeitaufwändiger und erfordert den Umgang mit stark ätzenden Flüssigkeiten.

Es besteht daher ein Bedarf an verbesserten analytischen Verfahren und Vorrichtungen zur Bestimmung der Phosphat-Konzentration mit hoher Empfindlichkeit.

Aufgabe der Erfindung ist es daher, eine gattungsgemäße Testvorrichtung bzw. ein Testverfahren zur Bestimmung der Phosphat-Konzentration dahingehend zu verbessern, dass sie bezüglich mindestens einer der oben genannten Nachteile verbessert wird.

## Zusammenfassung der Erfindung

Diese Aufgabe wird gemäß der Erfindung dadurch gelöst, dass eine Testvorrichtung zur Bestimmung der Konzentration von anorganischem Phosphat in einer flüssigen Probe bereitgestellt wird, wobei die Testvorrichtung eine Trägermatrix enthält, die als Träger für die flüssige Probe, die Nachweisreagenzien und auch die Nachweisreaktion dient, und wobei die Trägermatrix Folgendes umfasst:

a) Mindestens ein Molybdatsalz;
b) mindestens eine Säure, die bei Raumtemperatur fest ist;
c) ein Polyvinylpyrrolidon-Derivat, wobei das PVP-Derivat ein PVP mit einem K-Wert von 40 ist;
d) eine der folgenden Kombinationen von zwei chromogenen Reduktionsmitteln:

(i) Leuko Bindschedlers Grün (LBG) und 3,3',5,5'-Tetramethylbenzidin (TMB),
(ii) LBG und 4,4'-Methylen-N,N-bis-dimethylanilin (MDA),
(iii) LBG und Leuko Malachitgrün (LMG) oder
(iv) LBG und Leuko Kristallviolett (LKV).

Die bevorzugten Ausführungsformen der vorliegenden Erfindung ergeben sich aus den anhängigen Ansprüchen.

Die erfindungsgemäße Testvorrichtung vereinigt mehrere entscheidende Vorteile gegenüber den aus dem Stand der Technik bekannten Orthophosphat-Testvorrichtungen.

Wie die Erfinder festgestellt haben, führen die genannten Kombinationen aus zwei unterschiedlichen chromogenen Reduktionsmitteln zu einer signifikanten Verbesserung der Nachweisgrenze. So kann das Orthophosphat bereits in einer Konzentration von 0,25 mg/L nachgewiesen werden.

Damit werden geringe Konzentrationen, wie sie für viele Anwendungen (wie beispielsweise Aquariumwasser-Tests) relevant sind, in eindeutiger Weise detektierbar.

Da die Testvorrichtung auf einer schnellen Redox-Reaktion mit direktem Farbnachweis beruht, stellt sie ein direktes und sehr schnelles Nachweisverfahren dar (sog. "Schnelltest"). Zudem ist die erfindungsgemäße Testvorrichtung einfach zu gebrauchen und zu interpretieren und bedarf keiner zusätzlichen Messgeräte. Dies erlaubt eine Anwendung gerade im privaten Bereich, da auch ein Benutzer ohne technische oder laboranalytische Ausbildung einen raschen und zuverlässigen Sichtnachweis durchführen kann.

Bei der erfindungsgemäßen Vorrichtung ist auch, im Gegensatz zu vielen anderen Orthophosphat-Testsystemen, kein Zusatzreagenz notwendig, das vorher mit der zu messenden Probe vermischt werden muss, um dann dieses Gemisch mit der eigentlichen Testvorrichtung zu vermessen. Bei der erfindungsgemäßen Vorrichtung kann die Orthophosphat-haltige Probe ohne Vorbehandlung direkt mit der Testvorrichtung vermessen werden.

Weiterhin vermag es die erfindungsgemäße Vorrichtung auch ohne Zugabe eines Inertfarbstoffes, die hohe Messempfindlichkeit zu erreichen.

Die Testvorrichtung erlaubt eine selektive, d.h. lediglich auf Orthophosphat ausgerichtete Bestimmung.

Durch entsprechende Vorbehandlung der Probe kann der Nachweis auch auf andere Phosphatspezies wie organische Phosphate, Diphosphate, Metaphosphate oder Polyphosphate ausgeweitet werden.

Die Testvorrichtung weist auch eine ausreichende Stabilität auf und bedarf bei Verwendung der herkömmlichen Reagenzien auch keiner Kühlung.

Durch die Auswahl geeigneter Inhaltsstoffe erlaubt die Robustheit der Testvorrichtung eine bestimmte Variabilität der zu analysierenden Probe (z.B. Probenvorbehandlung nicht erforderlich) und/oder anderer definierter physikalischer Parameter während des Messvorganges und liefert trotzdem reproduzierbare und standardisierbare Ergebnisse.

Sie erfüllt zudem somit alle Anforderungen, die eine analytisch eingesetzte Testvorrichtung bzw. ein Testverfahren erfüllen müssen, und ist somit auch validierbar.

Die Testvorrichtung kann mit handelsüblichen Substanzen auf einfache Weise und zudem kostengünstig hergestellt werden.

Die Testvorrichtung kann ohne Mehraufwand in die bereits etablierten Testsysteme integriert werden.

## Ausführliche Beschreibung der Erfindung

Die Testvorrichtung basiert auf einer Ausbildung eines Molybdatophosphorsäure-Komplexes mit anschließender Reduktion unter Ausbildung von insgesamt drei farbigen Molekülspezies:

1. Molybdänblau,
2. Farbige oxidierte Form eines ersten chromogenen Reduktionsmittels, und

3. Farbige oxidierte Form eines zweiten chromogenen Reduktionsmittels.

Als Arbeitshypothese wird davon ausgegangen, dass es die Ausbildung von insgesamt drei farbigen Molekülspezies ist, die hier zu einem besonders empfindlichen Messverfahren führen.

Die Anfärbung der Testmatrix kann mit unterschiedlichen Methoden ausgewertet werden. In der einfachsten Form wird sie mit einer entsprechenden Farbe auf einer Standardfarbkarte verglichen, bei der die einzelnen Farbwerte bestimmten PhosphatKonzentrationen zugeordnet sind.

In einer weiteren Ausführungsform sieht die Testvorrichtung die Verwendung mindestens eines Inertfarbstoffs vor. Dieser Inertfarbstoff ist auf das chromogene Reduktionsmittel oder deren Kombination mit ihrem Farbumschlag abgestimmt und erlaubt durch eine gleichbleibende Hintergrundfärbung eine bessere Detektion des Farbumschlags. So ist bei einer Veränderung von Farblos nach Grün die Verwendung eines gelborangen Inertfarbstoffs vorteilhaft.

Der Inertfarbstoff ist erfindungsgemäß im gleichen Abschnitt wie das chromogene Reduktionsmittel bereitzustellen. Dies kann in mehrfacher Hinsicht realisiert werden, wie bspw.:

(a) Der Inertfarbstoff kann auf dem Träger selber aufgebracht sein, bzw. durch einen gefärbten Träger bereitgestellt werden.
(b) Der Inertfarbstoff kann mit in die Trägermatrix eingebracht werden.
(c) Die Trägermatrix selber kann farbig ausgestaltet sein.
(d) Die Testvorrichtung kann unter der Trägermatrix noch eine separate gefärbte Schicht aufweisen.

In bevorzugter Weise wird genau ein Inertfarbstoff eingesetzt. Es können allerdings auch zwei, drei oder noch mehr Inertfarbstoffe eingesetzt werden, um hier eine für das System optimale Farbigkeit zu erzielen.

In weiterhin bevorzugter Weise enthält hierbei die Trägermatrix genau einen Inertfarbstoff.

In einer Ausführungsform der Erfindung ist mindestens ein Inertfarbstoff ausgewählt aus der Gruppe enthaltend Tartrazin, Neozapongelb, Ponceau S, Gelborange, und Nitrazingelb, wobei der Inertfarbstoff bevorzugt Gelborange ist.

In einer alternativen ebenso bevorzugten Ausführungsform umfasst die Trägermatrix keinen Inertfarbstoff. Wie oben dargelegt, erlaubt die erfindungsgemäße Vorrichtung bereits durch ihre Kombination aus zwei unterschiedlichen chromogenen Reduktionsmitteln das Erreichen einer herausragenden Messempfindlichkeit. Ein Inertfarbstoff ist in der Grundausführung daher nicht notwendig.

Das in der Testmatrix enthaltene mindestens eine Molybdatsalz kann jegliches wasserlösliches Salz sein, insofern es mit den anderen Nachweisreagenzien und der Testmatrix selber kompatibel ist. Das Molybdatsalz ist hierbei bevorzugt ausgewählt aus der Gruppe bestehend aus Ammoniummolybdat, Natriummolybdat, Calciummolybdat und Kaliummolybdat, wobei Ammoniummolybdat besonders bevorzugt ist. Die Konzentration des Molybdatsalzes in der Imprägnierungslösung beträgt zwischen 2 und 50 g/L, bevorzugt zwischen 5 und 15 g/L und besonders bevorzugt zwischen 7 und 11 g/L.

In weiterhin bevorzugter Weise enthält hierbei die Trägermatrix genau ein Molybdatsalz.

Zur Ausbildung eines stark aziden pH-Werts enthält die Testmatrix auch mindestens eine feststoffliche Säure. Die feststoffliche Säure der Testvorrichtung dient der Komplexbildung zwischen Phosphat und Molybdat und erlaubt durch die Ausbildung eines stark aziden pH-Werts die Quantifizierung des Phosphats. Insofern sie als Feststoff vorliegt, verflüchtigt sie sich nicht, sondern verbleibt als Feststoff in der Trägermatrix und erlaubt eine langzeitstabile Formulierung.

Zweckmäßigerweise handelt es sich bei der Säure um eine organische Säure.

Die mindestens eine feststoffliche Säure ist hierbei bevorzugt eine organische Amidosulfonsäure.

In einer bevorzugten Ausführungsform ist die Säure ausgewählt aus der Gruppe bestehend aus Methylamidosulfonsäure, Amidoschwefelsäure und Cyclaminsäure, wobei die Cyclaminsäure bevorzugt ist.

In bevorzugter Weise wird genau eine Säure eingesetzt. Es können allerdings auch zwei, drei oder noch mehr Säuren eingesetzt werden, um bspw. Polyphosphate in der Ausgangslösung unter Hydrolyse zur Orthophosphat aufzuschließen.

In einer Ausführungsform der Erfindung liegt die feststoffliche Säure als reine Säure vor, wird also nicht durch Zugabe ihrer konjugierten Base zu einem Puffersystem ergänzt.

Erfindungsgemäß weist die Testvorrichtung ein Polyvinylpyrrolidon-Derivat auf, wobei das Polyvinylpyrrolidon-Derivat ein Polyvinylpyrrolidon mit einem K-Wert von 40 ist und als Katalysator und als Netzmittel fungiert. Das in der Testmatrix enthaltene Polyvinylpyrrolidon kann jegliches wasserlösliche PVP-Derivat sein, insofern es mit den anderen Nachweisreagenzien und der Testmatrix selber kompatibel ist. Die Konzentration des Polyvinylpyrrolidons in der Imprägnierungslösung beträgt zwischen 20 und 120 g/L, bevorzugt zwischen 30 und 80 g/L und besonders bevorzugt zwischen 40 und 60 g/L.

Erfindungsgemäß werden bei der Testvorrichtung die genannten Kombinationen von zwei chromogenen Reduktionsmitteln verwendet, wobei diese Reduktionsmittel in ihrer reduzierten Leuko-Form eingesetzt werden, so dass sie in der Redoxreaktion zur oxidierten farbigen Form umgesetzt werden. Die Konzentration des jeweiligen chromogenen Reduktionsmittels in der Imprägnierungslösung beträgt zwischen 0,25 und 5 g/L, bevorzugt zwischen 0,5 und 2,5 g/L und besonders bevorzugt zwischen 0,7 und 2 g/L.

Das LBG liegt hierbei in der Imprägnierlösung bevorzugt in einer Konzentration zwischen 0,1 und 2,0 g/L, besonders bevorzugt zwischen 0,2 und 1,8 g/L und insbesondere zwischen 0,5 und 0,7 g/L vor.

In der Erfindung weist die Testmatrix der Testvorrichtung eine Kombination von genau zwei chromogenen Reduktionsmitteln auf, die eine der folgenden Kombinationen ist:

a) LBG und TMB
b) LBG und MDA
c) LBG und LMG, oder
d) LBG und LKV.

In einer speziellen Ausführungsform weist die Testmatrix eine Kombination aus LBG und TMB auf. Hierbei beträgt das molare Verhältnis von LBG zu TMB zwischen 10:1 und 1:10, bevorzugt zwischen 5:1 und 1:2 und ist besonders bevorzugt 2:1.

In einer weiteren Ausführungsform weist die Trägermatrix auch mindestens ein Tensid auf, um die Benetzbarkeit der Testmatrix zu erleichtern. In einer besonderen Ausführungsform wird hier eine Kombination aus einem nichtionischen und einem anionischen Tensid eingesetzt.

In einer Ausführungsform ist das mindestens eine nichtionische Tensid ausgewählt aus der Gruppe enthaltend Fettalkoholethoxylate (FAEO) wie Brij35, Fettalkoholpropoxylate (FAPO), Alkylglucoside wie Tween20, Alkylpolyglucoside (APG), Octylphenolethoxylate und Nonidet P40. In einer bevorzugten Ausführungsform ist das nichtionische Tensid Nonidet P40.

In einer Ausführungsform ist das anionische Tensid ausgewählt aus der Gruppe enthaltend Natriumdodecylsulfat, Ammoniumdodecylsulfat, Natriumlaurylethersulfat (SLES), Natriummyristylethersulfat, Natriumdioctylsulfosuccinat, Perfluoroctansulfonat (PFOS), Perfluorbutansulfonat und lineare Alkylbenzolsulfonate. In einer bevorzugten Ausführungsform ist das anionische Tensid Natriumdodecylsulfat.

In einer Ausführungsform liegt ein Nachweisreagenz, oder mehrere Nachweisreagenzien oder sogar alle Nachweisreagenzien als in der Trägermatrix immobilisiert vor.

In einer Ausführungsform der Erfindung ist die Testvorrichtung als Teststreifen oder Testband ausgebildet und/oder so ausgestaltet, dass sie in ein integriertes Testsystem aufnehmbar ist.

Der Teststreifen kann aus einer Vielzahl von Materialien hergestellt werden. Bevorzugt sind hierbei wasserfeste Materialien wie Kunststoffe. Der Teststreifen besteht hierbei bevorzugt aus Polyvinylchlorid oder Polyethylen.

In einer weiteren Ausführungsform kann die Testvorrichtung weitere Trägermatrizes aufweisen.

Durch eine oder mehrere zusätzliche Trägermatrizes, die ebenfalls zur Bestimmung der Phosphatkonzentration ausgerichtet sind, können unterschiedliche Messbereiche abgedeckt werden.

Zweckmäßigerweise können die zusätzlichen Trägermatrizes auch zur Bestimmung anderer Zielanalyten ausgestaltet sein. So ist es gerade im Aquariumbereich üblich, weitere Parameter zu ermitteln wie bspw. pH-Wert, Nitratgehalt, Nitritgehalt, Carbonathärte, Gesamthärte und Chlorgehalt.

Die erfindungsgemäße Trägermatrix ist zweckmäßigerweise aus einem flüssigkeitsabsorbierenden Material aufgebaut. Durch diese Eigenschaft nimmt das Material eine definierte Menge an zu analysierender Flüssigkeit auf und hält sie fest und erlaubt somit die Solubilisierung der feststofflichen Nachweisreagenzien durch die Flüssigkeit und die anschließende Nachweisreaktion in der Trägermatrix.

Dem Fachmann sind aus dem Stand der Technik zahlreiche Materialien und Strukturen bekannt, die als Trägermatrix geeignet sind und er kann in Abhängigkeit vom Testansatz diese gezielt auswählen.

In bevorzugter Weise ist die Trägermatrix ein flüssigkeitsabsorbierendes Material. Dieses Material ist bevorzugt ausgewählt aus der Gruppe enthaltend Filterpapier, Vlies, Glasfaser, poröses Polymermaterial aus Polysulfon, Polyester, Nylon, Nitrocellulose, PVDF, Polycarbonat, wobei ein Vlies oder Vliesstoff besonders bevorzugt ist.

In einer bevorzugten Ausführungsform handelt es sich bei der Trägermatrix um einen Vliesstoff. Vliesstoffe sind kostengünstig und stark saugfähig und können in einfacher Weise (durch Tränken und anschließendes Trocknen) mit den Testreagenzien imprägniert werden.

Das in der erfindungsgemäßen Vorrichtung verwendete Vlies kann aus jeder möglichen Art von Fasern aufgebaut sein. Bekannte Faserarten sind mineralische Fasern wie Glas, Asbest, Mineralwolle, tierische Fasern wie Seide und Wolle, pflanzliche Fasern wie Baumwolle und chemische Fasern. Bei den chemischen Fasern wird zwischen Fasern aus natürlichen Polymeren wie Cellulose und Fasern aus synthetischen Polymeren unterschieden. Letztere werden erfindungsgemäß auch als Kunststofffasern bezeichnet. Beispiele für chemische Fasern aus synthetischen Polymeren sind die Polyamide PA 6.6 (Nylon®), PA 6.0 (Perlon®), Polyester (PES) wie Polyethylenterephthalat (PET) und Polybutylenterephthalat (PBT), Polyvinylchlorid (PVC), Polypropylen (PP), Polyethylen (PE), Polyimid (PI), Polyamidimid (PAI), Polyphenylensulfid (PPS), Aramid, Polyacrylnitril (PAN), Polytetrafluorethylen (PTFE).

Für die Verbindungen der Fasern des Vlieses zu einem Vliesstoff sind dem Fachmann verschiedene Verfahren bekannt. Dazu zählen mechanische Verfahren wie Vernadelung oder Wasserstrahlverfestigung, chemische Verfahren, etwa durch die Zugabe von Bindemitteln oder thermische Verfahren, z. B. Erweichen in einem geeigneten Gasstrom, zwischen beheizten Walzen oder auch in einem Dampfstrom. Bevorzugt wird das Vlies mechanisch (z. B. durch Vernadelung als Nadelvliesstoff) oder thermisch verfestigt, oder die Verfestigung erfolgt durch eine Kombination aus mechanischem und thermischen Verfahren. Es hat sich gezeigt, dass mechanisch und/oder thermisch verfestigte Vliesstoffe eine bessere Farbreaktion zeigen als die chemisch oder chemisch und thermisch verfestigten Vliesstoffe, was durch deren schlechteres Saugverhalten bedingt sein mag.

Gemäß einer bevorzugten Ausführungsform weist der Vliesstoff einen Wasseraufnahmekoeffizienten von 500 bis 1000 kg/m²√h und besonders bevorzugt von 700 bis 900 kg/m²√h auf.

In einer speziellen Ausführungsform besteht das Vlies (oder entsprechend der verfestigte Vliesstoff) aus Polyester (PES) und/oder Viskose (CV). Wie in dem Ausführungsbeispiel 3 dargelegt, sind diese Materialien besonders vorteilhaft hinsichtlich hoher Messempfindlichkeit.

In einer Ausführungsform ist die Trägermatrix einschichtig aufgebaut, so dass alle Nachweisreagenzien in dieser Schicht enthalten sind. In einer alternativen Ausführungsform kann die Trägermatrix aus zwei oder mehr Schichten aufgebaut sein. Die einzelnen Schichten können so beispielsweise eine unterschiedliche Saugfähigkeit bzw. Aufnahmekapazität für Flüssigkeiten aufweisen, so dass die flüssige Probe gezielter aufgenommen werden kann und auch ein Ausbluten der Trägermatrix verhindert werden kann. Zudem erlaubt dies eine räumliche Trennung der unterschiedlichen Nachweisreagenzien, so dass chemisch/physikalisch nicht-kompatible Nachweisreagenzien verwendet werden können, oder die von außen eindringende flüssige Probe beim Durchdringen der einzelnen Schichten sequentiell mit den Nachweisreagenzien reagieren kann.

Weiterhin kann die Trägermatrix auch einen als "Waste-Pad" ("Abfall-Pad") bezeichneten Bereich aufweisen, der die durch die Trägermatrix durchgelaufene Flüssigkeit aufnimmt. In diesem Bereich kann z. B. eine saugfähige Matte, bzw. ein Vlies, ein Lösch- oder Filterpapier oder dergleichen vorgesehen sein.

In einer besonderen Ausführungsform stellt die Erfindung eine Testvorrichtung zur Bestimmung der Orthophosphat-Konzentration bereit, wobei die Trägermatrix Folgendes umfasst:

    a) Cyclaminsäure
    b) PVP K40
    c) Ammoniummolybdat
    d) Leuko Bindschedlers Grün
    e) 3,3',5,5'-Tetramethylbenzidin

In einer besonders bevorzugten Ausführungsform ist die vorgenannte Trägermatrix ein Vliesstoff, der auf einem Teststreifen aufgebracht ist.

In einem zweiten Aspekt stellt die Erfindung ein Testverfahren zur Bestimmung der Orthophosphat-Konzentration in einer flüssigen Probe unter Verwendung der erfindungsgemäßen Testvorrichtung bereit, wobei das Testverfahren die folgenden Schritte umfasst:

    a) Tränken der Trägermatrix mit der flüssigen Probe, bevorzugt durch Eintauchen in die Probe;
    b) Entfernen überschüssigen Probenmaterials von der Trägermatrix, bevorzugt durch Herausziehen des Teststreifens aus der flüssigen Probe; und optionales Abschütteln oder Abstreifen überschüssiger flüssiger Probe;
    c) Optional eine Inkubation des Teststreifens für mindestens 5 Sekunden, bevorzugt bei Raumtemperatur;
    d) Visuelles Erfassen des Farbwertes der Trägermatrix und bevorzugt unter Vergleich mit einem Farbstandard.

In einer bevorzugten Ausführungsform ist diese flüssige Probe eine wässrige Lösung.

In einer Ausführungsform kann die visuelle Erfassung des Farbwertes der Trägermatrix mit Hilfe eines Auswertungsgerätes erfolgen. Dieses Gerät kann ein Spektrometer sein, oder auch nur eine Vorrichtung mit einer Kamera. Hierbei ist ein mobiles Gerät mit einer Kamera bevorzugt. Solche mobilen Geräte sind beispielsweise Smartphones, Tablet-PCs, Digitalkameras angeschlossen an einen Computer oder kleine portable Computer mit eingebauter Kamera. In einer weiterhin bevorzugten Ausführungsform ist auf dem Gerät eine Applikation zur Aufnahme und Auswertung des Farbwertes gespeichert und dort auch ausführbar. Ein entsprechendes Messsystem zur Auswertung kolorimetrischer Assays ist beispielsweise in der deutschen Anmeldung DE 10 2016 202 428.0 offenbart.

In einem dritten Aspekt betrifft die Erfindung die Verwendung einer der erfindungsgemäßen Kombinationen aus zwei unterschiedlichen chromogenen Reduktionsmitteln zur Steigerung der Sensitivität eines Trägermatrix-basierten und auf einer Redoxreaktion beruhenden Analyseverfahrens zur Bestimmung der Konzentration von anorganischem Phosphat in einer flüssigen Probe.

In diesem Aspekt sind die Kombinationen von zwei unterschiedlichen chromogenen Reduktionsmitteln wie in Anspruch 1 oder 7 definiert.

**Definitionen**

Unter einer "Testvorrichtung" werden im Rahmen der vorliegenden Erfindung alle trägergebundenen Tests für medizinische und nichtmedizinische Zwecke verstanden. Bei diesen trägergebundenen Tests sind Nachweisreagenzien in der Trägermatrix eines Trägers eingebettet, der mit der flüssigen Probe in Kontakt gebracht wird, wobei die Trägermatrix als Träger für das Untersuchungsmaterial und als Träger für die Nachweisreaktion dient. Die Reaktion von flüssiger Probe und Reagenzien führt bei Anwesenheit des Zielanalyten, also hier der Orthophosphat-Ionen zu einem Farbumschlag, welcher visuell oder mit Hilfe eines Geräts beispielsweise transmissionsphotometrisch, reflexionsphotometrisch oder fluoreszenzphotometrisch ausgewertet werden kann.

Gemäß der Erfindung werden unter dem mit der Testvorrichtung vermessenen "Phosphat" alle Phosphatspezies verstanden, die sich von der Orthophosphorsäure ableiten und damit neben der Orthophosphorsäure das Dihydrogenphosphat ($H_2PO_4^-$), das Hydrogenphosphat ($HPO_4^{2-}$), und das Phosphat ($PO_4^{3-}$) umfassen.

Unter einem "chromogenen Reduktionsmittel" wird ein Reduktionsmittel verstanden, das in seiner reduzierten Form farblos oder nahezu farblos (cremefarben, hellgelb) ist und durch die Redoxreaktion in eine farbige oxidierte Form übergeht.

Gemäß der Erfindung ist unter einer "feststofflichen Säure" eine Säure zu verstehen, die bei Raumtemperatur in einem festen Aggregatzustand vorliegt. Dies kann sowohl eine organische Säure als auch eine anorganische Säure sein.

Der K-Wert stellt eine in der Kunststoffindustrie übliche Klassifikation dar und steht in direktem Zusammenhang mit der mittleren molaren Masse des Polymers. Damit lässt sich aus dem K-Wert indirekt auf den Grad der Polymerisation und damit die Kettenlänge schließen. Der auch als Eigenviskosität bezeichnete K-Wert wird über Viskositätsmessungen von Polymer-Lösungen bestimmt und häufig im technischen Bereich zur Bestimmung der molaren Masse von Polymeren, wie PVC, verwendet. Der K-Wert ist bei konstanten Messbedingungen, hinsichtlich Lösungsmittel, Lösungsmittelkonzentration und der Temperatur, nur abhängig von der mittleren molaren Masse der untersuchten Polymere. Er wird über die Beziehung K-Wert = 1000·k nach der Fikentscher-Gleichung berechnet, in der bedeuten: $\eta_r$ = relative Viskosität (dynamische Viskosität der Lösung/dynamische Viskosität des Lösungsmittels) und c = Massenkonzentration an Polymer in der Lösung in $g/cm^3$.

Fikentscher-Gleichung:

$$K = 1000 \cdot k = 1000 \cdot \frac{1{,}5 \lg \eta_r - 1 + \sqrt{1 + (\frac{2}{c} + 2 + 1{,}5 \lg \eta_r) \cdot 1{,}5 \lg \eta_r}}{150 + 300c}$$

Die Bestimmung des K-Werts erfolgt anhand der Norm DIN ISO 1628-1.

Erfindungsgemäß ist unter einem "Inertfarbstoff" ein Farbstoff zu verstehen, der zumindest im testgemäßen pH-Bereich (also im stark Sauren) seine Farbe nicht oder nur unwesentlich ändert und somit einen gleichbleibenden farbkontrastierenden Hintergrund ermöglicht.

Erfindungsgemäß wird unter einem "Vlies" ein Gebilde aus Fasern begrenzter Länge, Endlosfasern (Filamenten) oder geschnittenen Garnen jeglicher Art und jeglichen Ursprungs verstanden, die auf irgendeine Weise zusammengefügt bzw. miteinander verbunden sind. Davon ausgeschlossen ist das Verkreuzen bzw. Verschlingen von Garnen, wie es beim Weben, Wirken oder Stricken geschieht. Ein Vlies mit verfestigten Fasern wird auch als Vliesstoff bezeichnet. Die erfindungsgemäßen Vliese sind flexible Flächengebilde, das heißt, sie sind biegsam, ihre Hauptstrukturelemente sind Fasern, insbesondere textile Fasern, und sie weisen eine vergleichsweise geringe Dicke gegenüber ihrer Länge und Breite auf.

Erfindungsgemäß schließt der Begriff "umfassen", außer seiner wörtlichen Bedeutung, auch die Ausdrücke "bestehen im Wesentlichen aus" und "bestehen aus" mit ein. Somit kann Gegenstand, der speziell aufgelisteten Elemente "umfasst" neben diesen Elementen weitere Elemente enthalten oder im in Sinne von "bestehen aus" keine weiteren Elemente enthalten.

Unter "Selektivität" versteht man die Fähigkeit bestimmter Substanzen, aus einer Anzahl gebotener Möglichkeiten zur Reaktion eine bevorzugt auszusuchen. Die ausschließliche Auswahl bezeichnet man als Spezifität.

Als "Sensitivität" (Empfindlichkeit) bezeichnet man die Stärke der Änderung in der Antwort eines Messsignals geteilt durch die Änderung der auslösenden Größe (z. B. der Zielanalytkonzentration). Die Empfindlichkeit einer analytischen Methode entspricht der Steigung der Kalibrierkurve.

Erfindungsgemäß sind die zu unterscheidenden Begriffe der "Systempräzision" (Messpräzision) und der "Methodenpräzision" wie folgt definiert: Die Messpräzision ist ein Maß für die Schwankungen, die durch die Testvorrichtung oder dem damit arbeitenden Analysengerät selbst verursacht werden. Sie wird durch die Mehrfachanalyse (z. B. sechsfach) eines Standards ermittelt. Die Forderung an die Messpräzision hängt vom Analysengerät ab. Dagegen beschreibt die Methodenpräzision die zufällige Streuung der Analysenergebnisse. Sie wird durch eine mehrfache (meist sechsfache) Durchführung der gesamten Analyse, d. h. vom Abwiegen über die Probenvorbereitung bis zu der Messung und Befund ermittelt (sechs Einwaagen realer Proben).

Die "Stabilität" der Testvorrichtung beinhaltet Lagerstabilität, Stabilität unter physikalischen Einflüssen wie z. B. Wärme, Licht, mechanische Beanspruchung.

Die "Richtigkeit" ist ein Maß für die Abweichung des Messwertes vom richtigen Wert (manchmal als "wahrer" Wert bezeichnet) aufgrund eines systematischen Fehlers. Die Richtigkeit wird im Allgemeinen durch den Vergleich mit einem Referenz- oder Arbeitsstandard (Soll/Ist-Vergleich), den Vergleich mit einer unabhängigen, möglichst validierten Methode oder durch das sogenannte Aufstocken ("Spiken" einer Probe) bestimmt. Wenn bei bestimmten Proben keine der drei Methoden anwendbar ist, kann als Kriterium für die Richtigkeit folgendes gelten: Die Selektivität ist erwiesen, Linearität ist vorhanden, und die Kalibriergerade geht durch den Nullpunkt.

Die "Nachweisgrenze" ist die kleinste Konzentration (Menge) des Analyten in der Probe, die qualitativ noch erfasst werden kann (Ja/Nein-Entscheidung). Die "Bestimmungsgrenze" ist die kleinste Konzentration (Menge) des Analyten in der Probe, die mit gegebener Präzision und Richtigkeit quantitativ bestimmt werden kann. Das zugrunde liegende mathematische Modell und die Bestimmungsmethoden sind in der DIN 32645 beschrieben.

Die "Erfassungsgrenze" gibt die Konzentration (Menge) an, die mit einer Wahrscheinlichkeit von 50 % nachgewiesen werden kann. Somit kann die Erfassungsgrenze vereinfacht als die doppelte Nachweisgrenze angesehen werden.

## Ausführungsbeispiele

### 1. Herstellung einer Phosphat-Testvorrichtung

Der zu schützende Teststreifen kann wie folgt hergestellt werden. Es werden drei verschiedene Imprägnierlösungen angesetzt. Für einen Liter Imprägnierlösung 1 werden zunächst 72 g PVP K40 und 24 g Ammoniummolybdat in 800 mL vollentsalztem Wasser (VE-Wasser) gelöst. Anschließend werden unter starkem Rühren 200 mL Ethanol zugegeben. Für die zweite Lösung werden 57 g Cyclaminsäure in einem Liter VE-Wasser gelöst. Zur Herstellung der dritten Lösung, werden 0,9 g LBG und 1,8 g TMB in einem Liter Ethanol gelöst.

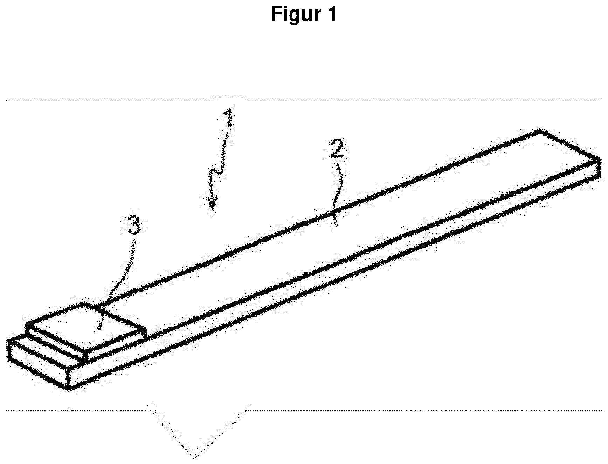

Zur Imprägnierung wird der entsprechende Vliesstoff in die jeweilige Testlösung eingetaucht und anschließend bei durchschnittlich 325 °C für etwa eine Minute getrocknet. Nach dem Zurechtschneiden des imprägnierten Vliesstoffes kann mit Hilfe des so erhaltenen Testfeldes der Teststreifen hergestellt werden (s. Fig. 1). Dazu wird das Testfeld mittels Schmelzkleber auf einen 5,5 x 95 mm großen PVC-Streifen befestigt und anschließend trocken und dunkel gelagert.

### 2. Vergleichender Test einer Phosphat-haltigen Testlösung

Um die Sensitivität der Testvorrichtung im Vergleich zu Testvorrichtungen aus dem Stand der Technik zu bestimmen wurde die folgenden fünf Teststreifen vergleichend untersucht:

1. AquaChek® Phosphate (Hach Company, Loveland, USA)
2. MQuant™ Phosphat-Test (Fa. Merck KGaA, Darmstadt, BRD)
3. Reflectoquant® plus Phosphat-Test (Fa. Merck KGaA, Darmstadt, BRD)
4. QUANTOFIX® Phosphat (Fa. MACHEREY-NAGEL, Düren, BRD)
5. Erfindungsgemäßer Teststreifen gemäß Beispiel 1.

Die Teststreifen wurden entsprechend der jeweiligen Testvorschrift in Lösungen mit einem Orthophosphatgehalt von 0,25; 0,5; 1,0; 3,0 und 5,0 mg/L und phosphatfreier Lösung eingetaucht und die Verfärbung der Teststreifen ermittelt.

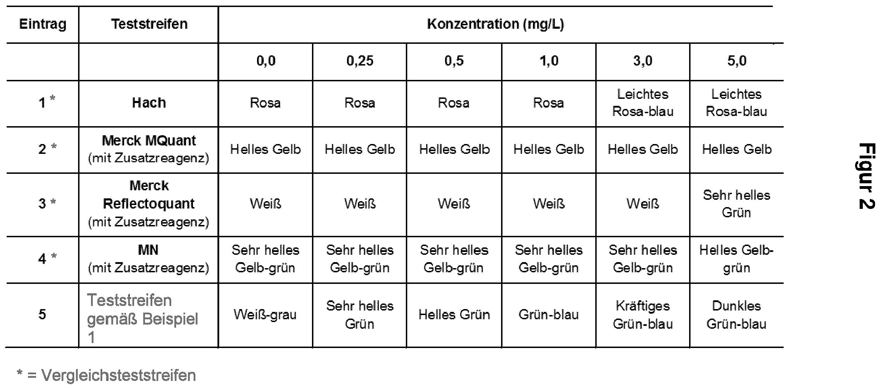

Die Tabelle in Fig. 2 fasst die Farbreaktionen der oben genannten Produkte im angestrebten Konzentrationsbereich zusammen und vergleicht die Farbreihen mit dem erfindungsgemäßen Teststreifen. Der Vergleich der fünf Teststreifen verdeutlicht, dass aktuelle Produkte (Einträge 1-4) die angestrebte Empfindlichkeit von <3 mg/L Phosphat nicht erreichen. Während beim MQuant™ Teststreifen von Merck (Eintrag 2) gar keine Farbreaktion zwischen 0 und 5 mg/L Phosphat hervorgerufen wird, zeigt der Reflectoquant® plus Phosphat-Test (Eintrag 3) eine Farbreaktion ab 5,0 mg/L Phosphat. Im Vergleich dazu zeigen sowohl die Teststreifen von Hach (Eintrag 1) als auch von MN mit Zusatzreagenz (Eintrag 4) ab 3 mg/L Phosphat eine Farbreaktion. Der erfindungsgemäße Teststreifen kann Phosphat in einer Konzentration ab etwa 0,25 mg/L Phosphat nachweisen (Eintrag 5) und ist damit um einen Faktor 12 sensitiver als der nächstbessere Teststreifen AquaChek® von Hach Company.

## 3. Vergleichstest unter Variation des Materials für die Trägermatrix

Für die Entwicklung eines hochempfindlichen Phosphat-Teststreifens spielen Vliesstoffe als Trägermaterial eine wichtige Rolle. Es werden 15 verschiedene Vliesstoffe mit unterschiedlichen Zusammensetzungen, Gewichten und Verfestigungsmethoden getestet. Die Teststreifen wurden gemäß Beispiel 1 hergestellt und zur Testung in Lösungen mit einem Orthophosphatgehalt von 0,25; 0,5; 1,0; 3,0 und 5,0 mg/L und phosphatfreier Lösung eingetaucht und die Verfärbung der Teststreifen ermittelt.

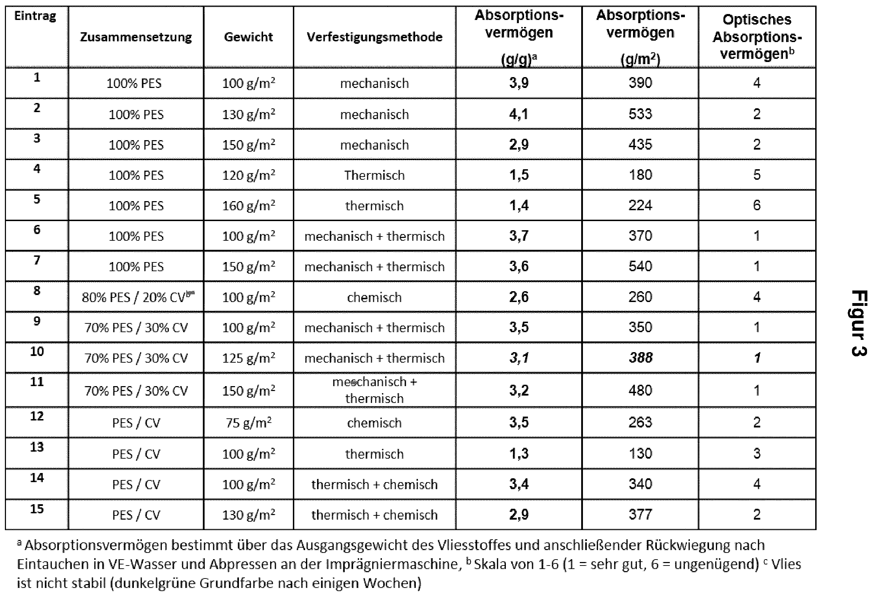

Alle Vliesstoffe unterscheiden sich deutlich in ihrem Gewicht (und damit einhergehende Dicke) sowie im Ausgangsmaterial. Dies bedingt das Saugverhalten und die damit verbundene Menge an Chemikalien, die auf das Trägermaterial aufgetragen werden. Grundsätzlich nehmen schwerere Vliesstoffe bei der Imprägnierung mehr Substanz auf, was einen hoch empfindlichen Test begünstigt. Die Haptik des Trägermaterials wird u.a. durch das Verfestigungsprinzip bestimmt. Mechanisch bzw. thermisch verfestigte Vliesstoffe (Einträge 1-5 und 13) und mechanisch und thermisch verfestigte Vliesstoffe zeigen zumeist eine gute Farbreaktion (Einträge 6, 7, 9-11). Rein chemisch (Einträge 8 und 12) und die thermisch und chemisch verfestigten Vliesstoffe hingegen zeigen oft ein schlechtes Saugverhalten und damit schlechte Farbreaktion. Die Tabelle in Figur 3 zeigt eine Übersicht der getesteten Vliesstoffe, hervorgehoben ist der Vliesstoff 10 (PES/CV mit 125 g/m$^2$) mit den besten Eigenschaften für den erfindungsgemäßen Teststreifen aus Beispiel 1.

Als relevanter Parameter wurde für die 15 Vliesstoffe das Absorptionsvermögen experimentell bestimmt (siehe Fig. 3). Das Absorptionsvermögen des Vlieses soll Aufschluss darüber geben, welche Menge an Reaktanden nach der Imprägnierung auf dem Vlies zurückbleiben. Durch Eintauchen in VE-Wasser und anschließendes Abpressen wird mittels der Gewichtsdifferenz das Absorptionsvermögen bestimmt. Da einige Vliesstoffe jedoch aus dem gleichen Material bestehen und sich nur im Gewicht unterscheiden, kann das Absorptionsvermögen auch auf die Fläche bezogen angegeben werden. Hier wird deutlich, dass ein bestimmtes Absorptionsvermögen (sowohl g/g als auch g/m$^2$) notwendig ist, um eine geeignete Farbreaktion zu ermöglichen. Dies liegt vor allem an zwei Faktoren. Zum einen muss genügend Menge an Imprägnierlösung auf das Vlies gebracht werden und zum anderen muss sich das Vlies mit der zu untersuchenden Lösung vollsaugen können, um eine Farbreaktion herrufen zu können. Vliesstoffe mit einem Absorptionsvermögen von <2 g/g (oder <250 g/m$^2$) sind deutlich zu hydrophob und somit für einen hochsensitiven Test ungeeignet (Einträge 4, 5 und 13). Alle Vliesstoffe mit einem Absorptionsvermögen von mindestens 3,0 g/g sind hydrophil genug, um nach der Imprägnierung eine Farbreaktion zu erhalten. Die angegebenen Vliesstoffe mit einem Absorptionsvermögen zwischen 2,0 und 3,0 g/g sind zwar saugfähig, aber für den Phosphat-Teststreifen nur bedingt geeignet. Dies liegt nicht nur an dem Absorptionsvermögen, sondern auch an anderen Parametern. Beispielsweise ist das Absorptionsvermögen von Vlies Nr. 8 mit 2,6 g/g recht hoch, doch durch eine chemische Verfestigung und den dadurch enthaltenen Binder, ist dieses Vlies schlechter als ein mechanisch und/oder thermisch verfestigtes Vlies. Gleiches gilt für Vlies Nr. 15. Das Vlies Nr. 10 liefert in den Versuchen die beste Farbreihe. Wie bereits bei den Herstellerangaben, sind alle Parameter im mittleren Bereich anzutreffen.

Das in der letzten Spalte der Tabelle angegebene optische Absorptionsvermögen ist eine Schätzung wie gut das Vlies Wasser aufsaugt. Alle Vliesstoffe werden gleich lange in Wasser eingetaucht. Hier bestätigt sich, dass die hydrophoben Vliese deutlich langsamer bis gar kein Wasser aufsaugen. Die Vliesstoffe, die optisch sehr gut aufsaugen, haben meist auch eine gute Farbreaktion im Versuch im kleinen Maßstab.

## 4. Vergleichstest unter Variation des PVP-Derivats

Wie in der Anmeldung beschrieben, wird die Testreaktion durch die Anwesenheit von Polyvinylpyrrolidon (kurz PVP) mit einem K-Wert von 40 optimiert. Das PVP fungiert einerseits als Netzmittel, andererseits ist es für die Umsetzung an sich wichtig.

Es werden 9 verschiedene PVP-Derivate und zudem ein Caprolactam getestet. Als Nullkontrolle wurde ein Teststreifen ohne PVP hergestellt (Nr. 1). Die Teststreifen wurden gemäß Beispiel 1 hergestellt und zur Testung in Lösungen mit einem Orthophosphatgehalt von 0,25; 0,5; 1,0; 3,0 und 5,0 mg/L und phosphatfreier Lösung eingetaucht und die Verfärbung der Teststreifen ermittelt.

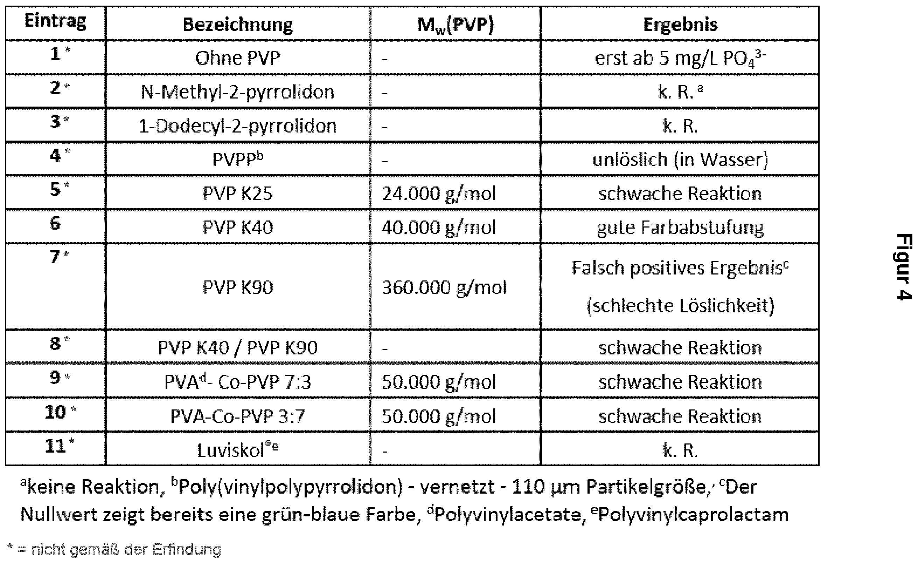

Die Tabelle in der Figur 4 zeigt die eingesetzten PVP-Derivate und die unterschiedlichen mittleren Molekulargewichte, die eingesetzt werden. Ohne den Einsatz von PVP wird zwar eine Farbreaktion erhalten, jedoch tritt diese erst bei etwa 5 mg/L Phosphat ein (Eintrag 1). Weitere Versuche zeigen, dass sowohl mit NMP (Eintrag 2) als auch mit 1-Dodecyl-2-pyrrolidon (Eintrag 3) keine Reaktion stattfindet. Während vernetztes PVP (Eintrag 4) nicht in Wasser löslich ist, zeigen alle anderen PVP-Varianten eine Farbreaktion. PVP mit einem mittleren Molekulargewicht von 24.000 g/mol zeigt zwar eine Farbreaktion (Eintrag 5), jedoch ist im niedrigen Konzentrationsbereich eine Differenzierung sehr schwierig. Wird ein Polymer mit einem mittleren Molekulargewicht von ca. 360.000 g/mol verwendet, wird bereits bei 0 mg/L Phosphat eine grün-blaue Farbe erhalten (Eintrag 7). Die Farbreihe bei PVP K40 ($M_w$ = 40.000 g/mol, Eintrag 6), welches gemäß der Erfindung ist, zeigt die beste Unterscheidbarkeit. Eine Mischung aus PVP K40 und K90 (Eintrag 8) liefert keine Verbesserung der Farbreihe. Die Copolymere PVA-Co-PVP (3:7 und 7:3,

Einträge 9 und 10) zeigen zwar eine Farbreaktion, jedoch ebenfalls schwächer als PVP K40. Bei Luviskol (Eintrag 11) handelt es sich um ein Caprolactam, es findet keine Farbreaktion statt.

**5. Vergleichstest unter Variation der feststofflichen Säure**

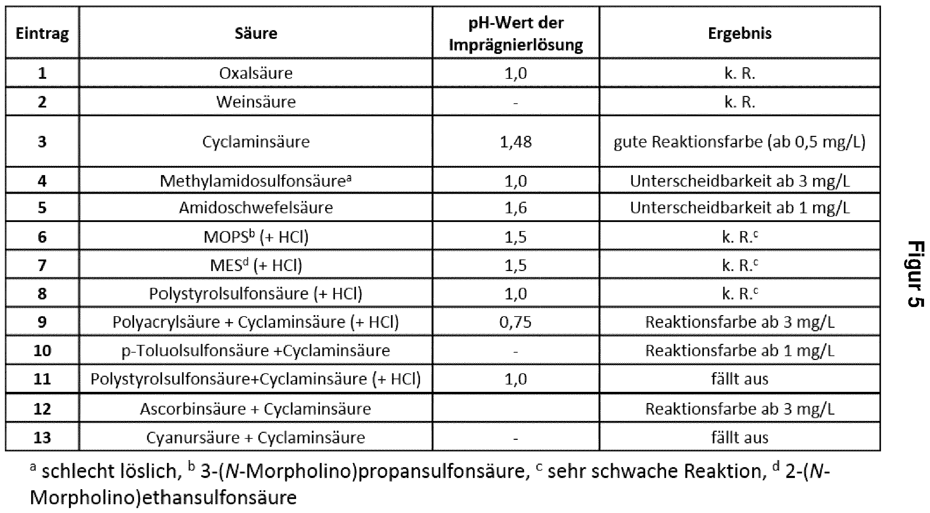

Für die Reaktion von Molybdatophosphorsäure zu Molybdänblau wird ein niedriger pH-Wert benötigt. Zur Ermittlung der optimalen Säure wurden 13 unterschiedliche Säuren getestet. Die Teststreifen wurden gemäß Beispiel 1 hergestellt und zur Testung in Lösungen mit einem Orthophosphatgehalt von 0,25; 0,5; 1,0; 3,0 und 5,0 mg/L und phosphatfreier Lösung eingetaucht und die Verfärbung der Teststreifen ermittelt. Die Ergebnisse sind in der Tabelle in Figur 5 wiedergegeben.

Es wurden verschiedene Säure-Klassen getestet. Die Carbonsäuren Oxalsäure und Weinsäure (Einträge 1 und 2) liefern keine Farbreaktion. Hingegen liefern Derivate der Amidoschwefelsäure eine Farbreaktion für die Bestimmung von Phosphat. Dabei zeigt das Testpapier mit Cyclaminsäure (Eintrag 3) die beste Farbreihe. Testpapiere, bei denen Methylamidoschwefelsäure (Eintrag 4) oder Amidoschwefelsäure (Eintrag 5) genutzt werden, zeigen eine niedrigere Empfindlichkeit als Cyclaminsäure (Farbreaktion ab 3 bzw. 1 mg/L Phosphat im Vergleich zu 0,5 mg/L Phosphat. Weiterhin zeigen Systeme mit MOPS, MES und Polystyrolsulfonsäure in Kombination mit Salzsäure nur eine sehr schwache Farbreaktion (Einträge 6-8). Auch die Kombination von Cyclaminsäuren mit anderen Säuren lieferte keine Verbesserung. Hierbei werden beispielsweise p-Toluolsulfonsäure (Eintrag 10) oder Polymere mit Säurefunktionalität, wie Polyacrylsäure (Eintrag 9), oder auch Polystyrolsulfonsäure (Eintrag 11) getestet. Säuren, welche auch als Reduktionsmittel fungieren können (i.e. Ascorbinsäure, Eintrag 12), werden ebenfalls getestet, jedoch ohne nennenswerten Erfolg. Cyanursäure in Kombination mit Cyclaminsäure (Eintrag 13) kann aufgrund von Löslichkeitsproblemen nicht getestet werden.

**6. Vergleichstest unter Variation der Reduktionsmittel**

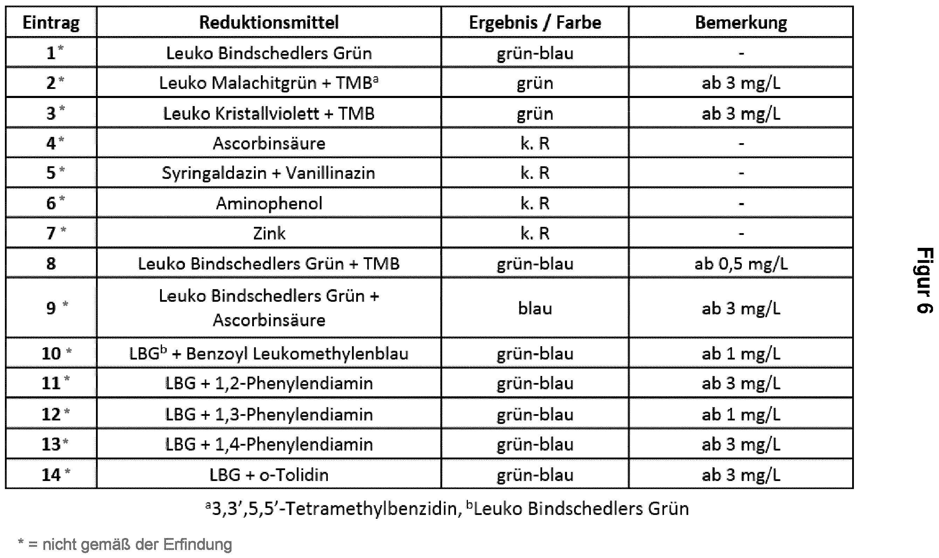

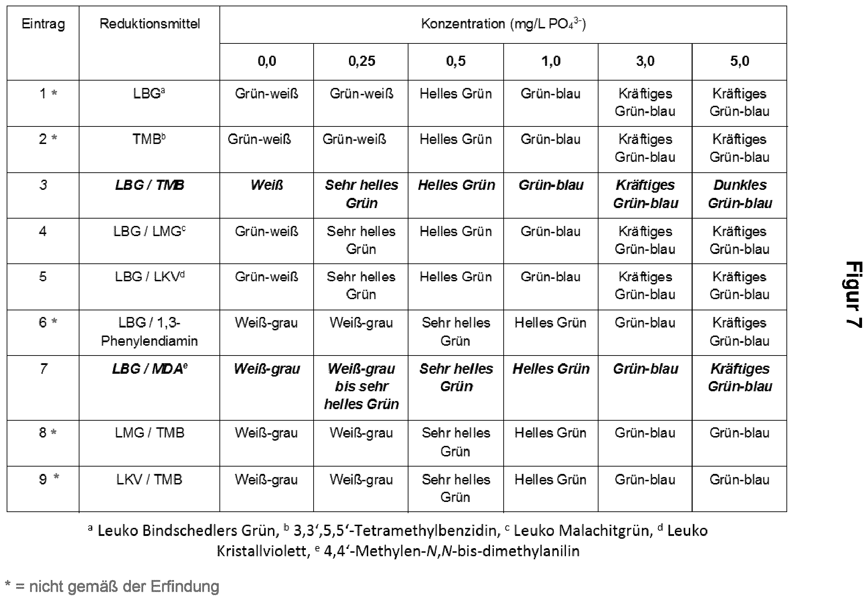

Für die Farbreaktion ist die Anwesenheit eines Reduktionsmittels unerlässlich. Zur Ermittlung des optimalen Reduktionsmittels bzw. der optimalen Reduktionsmittel-Kombination wurden in zwei Testreihen insgesamt 23 unterschiedliche Vorrichtungen hergestellt und getestet. Die Teststreifen wurden gemäß Beispiel 1 hergestellt und zur Testung in Lösungen mit einem Orthophosphatgehalt von 0,25; 0,5; 1,0; 3,0 und 5,0 mg/L und phosphatfreier Lösung eingetaucht und die Verfärbung der Teststreifen ermittelt. Die Ergebnisse sind in den Tabellen in Figur 6 und 7 wiedergegeben.

Die Tabelle in Figur 6 fasst die getesteten Reduktionsmittel der ersten Testreihe zusammen. Neben gängigen Reduktionsmitteln wie Ascorbinsäure (Eintrag 4), werden auch Leuko-Basen (Einträge 1-3) als chromogene Reduktionsmittel getestet, die nach Oxidation in ein Chromogen übergehen und somit die Farbreaktion verstärken sollen. Während Ascorbinsäure, Syringaldazin und Vanillinazin (Eintrag 5), Aminophenol (Eintrag 6) oder auch Zink (Eintrag 7) keine Farbreaktion hervorrufen, zeigen Leuko-Farbstoffe eine Farbreaktion, allen voran Leuko Bindschedlers Grün (LBG). Aufgrund der grünen Farbe des LBGs und der blauen Farbe von Molybdänblau, erscheint das Testfeld in Anwesenheit von Phosphat grün-blau. Weiterhin werden Kombinationen von LBG mit anderen Reduktionsmitteln getestet, um eine schnelle und vollständige Reduktion zu gewährleisten. In Kombination mit einem anderen Leuko-Farbstoff, wie beispielsweise Benzoyl Leuko Methylenblau (Eintrag 10), wird zwar eine Farbreaktion erhalten, jedoch kann mit der Kombination von LBG und TMB (Eintrag 8) das beste Ergebnis erhalten werden. Kombinationen mit Ascorbinsäure (Eintrag 9), Phenylendiaminen (Einträge 11-13) oder o-Tolidin (Eintrag 14) zeigen erst ab 1 bzw. 3 mg/L Phosphat eine Farbreihe. Eine Empfindlichkeit von bis zu 0,5 mg/L Phosphat ist möglich. Leuko Malachitgrün (Eintrag 2) und Leuko Kristallviolett (Eintrag 3) erzeugt ebenfalls mit TMB eine entsprechende Farbreaktion, aber auch diese ist schwächer als der Vergleich mit LBG und TMB.

Die Tabelle in Figur 7 fasst die getesteten Reduktionsmittel der zweiten Testreihe zusammen. Neben Leuko Bindschedlers Grün (LBG, Eintrag 1) liefert auch 3,3',5,5'-Tetramethylbenzidin (TMB, Eintrag 2) eine Farbreaktion. Die erfindungsgemäße Kombination beider Reduktionsmittel (Eintrag 3) liefert die beste Farbreaktion und die bestmögliche Farbabstufung im Konzentrationsbereich 0-5 mg/L Phosphat. Eine Empfindlichkeit von 0,25 mg/L Phosphat ist möglich. Die erfindungsgemäße Kombination von LBG mit anderen Leukobasen, wie beispielsweise Leuko Malachitgrün (Eintrag 4) oder Leuko Kristallviolett (Eintrag 5) liefern keine bessere Farbabstufung. LBG In Kombination mit 1,3-Phenylendiamin (Eintrag 6) liefert zwar eine Farbreaktion jedoch erst ab etwa 0,5 mg/L Phosphat. Wird 1,3-Phenylendiamin erfindungsgemäß durch 4,4'-Methylen-N,N-bis-dimethylanilin (MDA, Eintrag 7) ersetzt,

kann ebenfalls eine sehr gute Farbabstufung erreicht werden. Diese ist im Ganzen etwas heller als bei der Kombination von LBG und TMB.

Leuko Malachitgrün (Eintrag 8) und Leuko Kristallviolett (Eintrag 9) in Kombination mit TMB liefern ebenfalls eine Farbreihe. Die Empfindlichkeit liegt bei etwa 0,5 mg/L Phosphat, ab 3,0 mg/L Phosphat wird die Unterscheidung schwierig.

Es zeigen:

Fig. 1 zeigt eine als Teststreifen ausgebildete Testvorrichtung mit einem Kunststoffstreifen 2 als Träger, auf das eine Trägermatrix 3 befestigt ist. Die Trägermatrix enthält die für die Bestimmung notwendigen Nachweisreagenzien, die in bevorzugter Weise Cyclaminsäure, PVP K40, Ammoniummolybdat, Leuko Bindschedlers Grün und 3,3',5,5'-Tetramethylbenzidin ist.

Fig. 2 zeigt die Ergebnisse der Messung aus Beispiel 2 unter Vergleich der erfindungsgemäßen Testvorrichtung mit vier handelsüblichen Teststreifen. Dargestellt ist die visuelle erfasste Farbänderung der Teststreifen nach Eintauchen in einer Lösung mit 0,0; 0,25; 0,5; 1,0; 3,0 und 5,0 mg/L Phosphat.

Fig. 3 zeigt die Ergebnisse der Messung aus Beispiel 3 mit der erfindungsgemäßen Testvorrichtung unter Variation des Trägermatrix-Materials, wobei 15 unterschiedliche Vliesstoffe vergleichend getestet wurden.

Fig. 4 zeigt die Ergebnisse der Messung aus Beispiel 4 mit der erfindungsgemäßen Testvorrichtung unter Variation des PVP-Derivats, wobei 10 unterschiedliche Derivate vergleichend getestet wurden.

Fig. 5 zeigt die Ergebnisse der Messung aus Beispiel 5 mit der erfindungsgemäßen Testvorrichtung unter Variation der feststofflichen Säure, wobei 13 unterschiedliche Säuren bzw. Säurekombinationen vergleichend getestet wurden.

Fig. 6 zeigt die Ergebnisse der ersten Testreihe aus Beispiel 6 mit der erfindungsgemäßen Testvorrichtung unter Variation des Reduktionsmittels, wobei 14 unterschiedliche Reduktionsmittel oder Gemische hiervon vergleichend getestet wurden.

Fig. 7 zeigt die Ergebnisse der zweiten Testreihe aus Beispiel 6 mit der erfindungsgemäßen Testvorrichtung unter Variation des Reduktionsmittels, wobei 9 unterschiedliche Reduktionsmittel oder Gemische hiervon vergleichend getestet wurden.

## Bezugszeichenliste

1 Testvorrichtung
2 Kunststoffstäbchen
3 Trägermatrix

In den Patentansprüchen verwendete Begriffe wie "umfassen", "aufweisen", "beinhalten", "enthalten" und dergleichen schließen weitere Elemente oder Schritte nicht aus. Die Verwendung des unbestimmten Artikels schließt eine Mehrzahl nicht aus. Eine einzelne Einrichtung kann die Funktionen mehrerer in den Patentansprüchen genannten Einheiten bzw. Einrichtungen ausführen. In den Patentansprüchen angegebene Bezugszeichen sind nicht als Beschränkungen der eingesetzten Mittel und Schritte anzusehen.

## Patentansprüche

1. Testvorrichtung zur Bestimmung der Konzentration von anorganischem Phosphat in einer flüssigen Probe enthaltend eine Trägermatrix, die als Träger für die flüssige Probe, die Nachweisreagenzien und die Nachweisreaktion dient, wobei die Trägermatrix Folgendes umfasst:

    a) mindestens ein Molybdatsalz;
    b) mindestens eine Säure, die bei Raumtemperatur fest ist;
    c) ein Polyvinylpyrrolidon-Derivat, wobei das Polyvinylpyrrolidon-Derivat Polyvinylpyrrolidon mit einem K-Wert von 40 ist; und

d) eine der folgenden Kombinationen von zwei chromogenen Reduktionsmitteln:

(i) Leuko Bindschedlers Grün und 3,3',5,5'-Tetramethylbenzidin,
(ii) Leuko Bindschedlers Grün und 4,4'-Methylen-*N,N*-bis-dimethylanilin,
(iii) Leuko Bindschedlers Grün und Leuko Malachitgrün, oder
(iv) Leuko Bindschedlers Grün und Leuko Kristallviolett.

2. Testvorrichtung gemäß Anspruch 1, **dadurch gekennzeichnet, dass** das Molybdatsalz ausgewählt ist aus der Gruppe bestehend aus Ammoniummolybdat, Natriummolybdat, Calciummolybdat und Kaliummolybdat.

3. Testvorrichtung gemäß Anspruch 1 oder 2, **dadurch gekennzeichnet, dass** die mindestens eine feststoffliche Säure ausgewählt ist aus der Gruppe bestehend aus Methylamidosulfonsäure, Amidoschwefelsäure und Cyclaminsäure, wobei die Cyclaminsäure bevorzugt ist.

4. Testvorrichtung gemäß einem der vorangehenden Ansprüche, **dadurch gekennzeichnet, dass** die Trägermatrix ein flüssigkeitsabsorbierendes Material ist, und bevorzugt ausgewählt ist aus der Gruppe enthaltend Filterpapier, Vlies, Glasfaser, poröses Polymermaterial aus Polysulfon, Polyester, Nylon, Nitrocellulose, PVDF, Polycarbonat und besonders bevorzugt ein Vlies ist.

5. Testvorrichtung gemäß Anspruch 4, **dadurch gekennzeichnet, dass** das Vlies aus Polyester (PES) und/oder Viskose (CV) besteht.

6. Testvorrichtung gemäß Anspruch 4 oder 5, **dadurch gekennzeichnet, dass** das Vlies ein mechanisch und/oder thermisch verfestigter Vliesstoff ist, der bevorzugt einen Wasseraufnahmekoeffizienten von 500 bis 1000 kg/m$^2\sqrt{h}$ und besonders bevorzugt von 700 bis 900 kg/m$^2\sqrt{h}$ aufweist.

7. Testvorrichtung gemäß einem der vorangehenden Ansprüche zur Bestimmung der Phosphat-Konzentration, **dadurch gekennzeichnet, dass** sie ein Teststreifen mit einer Trägermatrix ist, die Folgendes umfasst:

(a) Cyclaminsäure
(b) PVP K40
(c) Ammoniummolybdat
(d) Leuko Bindschedlers Grün
(e) 3,3',5,5'-Tetramethylbenzidin.

8. Testvorrichtung gemäß einem der vorangehenden Ansprüche, **dadurch gekennzeichnet, dass** sie als Teststreifen, Teststäbchen oder Testband ausgebildet ist und/oder in ein integriertes Testsystem aufnehmbar ist.

9. Testverfahren zur Bestimmung der Orthophosphat-Konzentration in einer flüssigen Probe mit einer Testvorrichtung gemäß einem der vorangehenden Ansprüche, umfassend die folgenden Schritte:

(a) Tränken der Trägermatrix mit der flüssigen Probe, bevorzugt durch Eintauchen in die Probe;
(b) Entfernen überschüssigen Probenmaterials von der Trägermatrix, bevorzugt durch Herausziehen des Teststreifens aus der flüssigen Probe; und optionales Abschütteln oder Abstreifen überschüssiger flüssiger Probe;
(c) Optional eine Inkubation des Teststreifens für mindestens 5 Sekunden, bevorzugt bei Raumtemperatur;
(d) Visuelles Erfassen des Farbwertes der Trägermatrix und bevorzugt unter Vergleich mit einem Farbstandard.

10. Verwendung einer Kombination aus zwei unterschiedlichen chromogenen Reduktionsmitteln wie in Anspruch 1 oder 7 definiert, zur Steigerung der Sensitivität eines Trägermatrix-basierten und auf einer Redoxreaktion beruhenden Analyseverfahrens zur Bestimmung der Konzentration von anorganischem Phosphat in einer flüssigen Probe.

**Claims**

1. A test device for determining the concentration of inorganic phosphate in a liquid sample, wherein said test device contains a support matrix serving as a support for the liquid sample, the detection reagents and the detection reaction, wherein said support matrix comprises:

a) at least one molybdate salt;

b) at least one acid that is solid at room temperature;

c) a polyvinyl pyrrolidone derivative, wherein said polyvinyl pyrrolidone derivative is polyvinyl pyrrolidone having a K value of 40; and

d) one of the following combinations of two chromogenic reducing agents:

> (i) leuko Bindschedler's green and 3,3',5,5'-tetramethylbenzidine;
> (ii) leuko Bindschedler's green and 4,4'-methylene-N,N-bis(dimethylaniline);
> (iii) leuko Bindschedler's green and leuko malachite green; or
> (iv) leuko Bindschedler's green and leuko crystal violet.

2. The test device according to claim 1, **characterized in that** said molybdate salt is selected from the group consisting of ammonium molybdate, sodium molybdate, calcium molybdate, and potassium molybdate.

3. The test device according to claim 1 or 2, **characterized in that** said at least one solid acid is selected from the group consisting of methylamidosulfonic acid, amidosulfonic acid, and cyclamic acid, cyclamic acid being preferred.

4. The test device according to any of the preceding claims, **characterized in that** said support matrix is a liquid-absorbing material, preferably selected from the group containing filter paper, non-woven, glass fiber, porous polymer material made of polysulfone, polyester, nylon, nitrocellulose, PVDF, polycarbonate, a non-woven being particularly preferred.

5. The test device according to claim 4, **characterized in that** said non-woven consists of polyester (PES) and/or viscose (CV).

6. The test device according to claim 4 or 5, **characterized in that** said non-woven is a mechanically and/or thermally bonded non-woven fabric, preferably having a water absorption coefficient of from 500 to 1000 $kg/m^2\sqrt{h}$ and more preferably of from 700 to 900 $kg/m^2\sqrt{h}$.

7. The test device according to any of the preceding claims for determining the phosphate concentration, **characterized by** being a test strip with a support matrix that includes:

> a) cyclamic acid
> b) PVP K40
> c) ammonium molybdate
> d) leuko Bindschedler's green
> e) 3,3',5,5'-tetramethylbenzidine.

8. The test device according to any of the preceding claims, **characterized by** being designed as a test strip, test rod or test tape, and/or can be included in an integrated test system.

9. A test method for determining the concentration of orthophosphate in a liquid sample using the test device according to any of the preceding claims, comprising the following steps:

> a) soaking the support matrix with the liquid sample, preferably by dipping into the sample;
> b) removing any excess sample material from the support matrix, preferably by withdrawing the test strip from the liquid sample; and optionally shaking off or stripping off excess liquid sample;
> c) optionally incubating the test strip for at least 5 seconds, preferably at room temperature;
> d) visually detecting the color value of the support matrix, preferably by comparison with a color standard.

10. The use of a combination of two different chromogenic reducing agents as defined in claim 1 or 7, for enhancing the sensitivity of an analytical method based on a support matrix and on a redox reaction, for determining the concentration of inorganic phosphate in a liquid sample.

**Revendications**

1. Dispositif de test pour déterminer la concentration de phosphate inorganique dans un échantillon liquide contenant une matrice de support qui sert de support de l'échantillon liquide, des réactifs de détection et de la réaction de détection, dans lequel la matrice de support comprend :

a) au moins un sel de molybdate ;
b) au moins un acide qui est solide à la température ambiante ;
c) un dérivé de polyvinylpyrrolidone, le dérivé de polyvinylpyrrolidone étant de la polyvinylpyrrolidone ayant une valeur K de 40 ; et
d) l'une des combinaisons suivantes de deux agents réducteurs chromogènes :

(i) leucobase de vert de Bindschedler et 3,3',5,5'tétraméthylbenzidine,
(ii) leucobase de vert de Bindschedler et 4,4'-méthylène-N,N-bis-diméthylaniline,
(iii) leucobase de vert de Bindschedler et leucobase de vert de Malachite, ou
(iv) leucobase de vert de Bindschedler et leucobase du violet cristallisé.

2. Dispositif de test selon la revendication 1, **caractérisé en ce que** le sel de molybdate est choisi dans le groupe constitué du molybdate d'ammonium, du molybdate de sodium, du molybdate de calcium et du molybdate de potassium.

3. Dispositif de test selon la revendication 1 ou 2, **caractérisé en ce que** l'acide solide, au moins au nombre de un, est choisi dans le groupe constitué de l'acide méthylamidosulfonique, de l'acide amidosulfurique et de l'acide cyclamique, l'acide cyclamique étant préféré.

4. Dispositif de test selon l'une des revendications précédentes, **caractérisé en ce que** la matrice de support est un matériau absorbant les liquides, et est de préférence choisie dans le groupe comprenant un papier filtre, un non-tissé, une fibre de verre, une matériau polymère poreux en polysulfone, du polyester, du nylon, de la nitrocellulose, du PVDF, un polycarbonate, et est de façon particulièrement préférée un non-tissé.

5. Dispositif de test selon la revendication 4, **caractérisé en ce que** le non-tissé se compose de polyester (PES) et/ou de viscose (CV).

6. Dispositif de test selon la revendication 4 ou 5, **caractérisé en ce que** le non-tissé est un non-tissé lié mécaniquement et/ou thermiquement, qui présente de préférence un coefficient d'absorption d'eau de 500 à 1000 kg/m$^2$√h et de façon particulièrement préférée de 700 à 900 kg/m$^2$√h.

7. Dispositif de test selon l'une des revendications précédentes pour déterminer la concentration en phosphate, **caractérisé en ce qu'**il s'agit d'une bandelette de test à matrice de support, qui comprend :

(a) de l'acide cyclamique
(b) du PVP K40
(c) du molybdate d'ammonium
(d) une leucobase de vert de Bindschedler
(e) de la 3,3',5,5'-tétraméthylbenzidine.

8. Dispositif de test selon l'une des revendications précédentes, **caractérisé en ce qu'**il est réalisé sous forme de bandelette de test, de bâtonnet de test ou de bande de test et/ou qu'il peut être logé dans un système de test intégré.

9. Procédé de test pour déterminer la concentration en orthophosphate dans un échantillon liquide en utilisant un dispositif de test selon l'une des revendications précédentes, comprenant les étapes suivantes consistant à :

(a) imprégner la matrice de support avec l'échantillon liquide, de préférence en l'immergeant dans l'échantillon ;
(b) retirer le matériau d'échantillon en excès de la matrice de support, de préférence en retirant la bandelette de test hors de l'échantillon liquide ; et facultativement en secouant le dispositif ou en éliminant du dispositif l'excès d'échantillon liquide ;
(c) facultativement faire incuber la bandelette de test pendant au moins 5 secondes, de préférence à température ambiante ;
(d) détecter visuellement la valeur de couleur de la matrice de support, et de préférence par comparaison avec un standard de couleur.

**10.** Utilisation d'une combinaison de deux agents réducteurs chromogènes différents tels que définis à la revendication 1 ou 7 pour augmenter la sensibilité d'un procédé d'analyse, à base d'une matrice de support et reposant sur une réaction d'oxydoréduction, permettant de déterminer la concentration en phosphate inorganique dans un échantillon liquide.

# Figur 1

| Eintrag | Teststreifen | Konzentration (mg/L) | | | | | |
|---|---|---|---|---|---|---|---|
| | | 0,0 | 0,25 | 0,5 | 1,0 | 3,0 | 5,0 |
| 1 * | Hach | Rosa | Rosa | Rosa | Rosa | Leichtes Rosa-blau | Leichtes Rosa-blau |
| 2 * | Merck MQuant (mit Zusatzreagenz) | Helles Gelb | Helles Gelb | Helles Gelb | Helles Gelb | Helles Gelb | Helles Gelb |
| 3 * | Merck Reflectoquant (mit Zusatzreagenz) | Weiß | Weiß | Weiß | Weiß | Weiß | Sehr helles Grün |
| 4 * | MN (mit Zusatzreagenz) | Sehr helles Gelb-grün | Sehr helles Gelb-grün | Sehr helles Gelb-grün | Sehr helles Gelb-grün | Sehr helles Gelb-grün | Helles Gelb-grün |
| 5 | Teststreifen gemäß Beispiel 1 | Weiß-grau | Sehr helles Grün | Helles Grün | Grün-blau | Kräftiges Grün-blau | Dunkles Grün-blau |

* = Vergleichsteststreifen

**Figur 2**

EP 3 578 991 B1

# Figur 3

| Eintrag | Zusammensetzung | Gewicht | Verfestigungsmethode | Absorptionsvermögen (g/g)[a] | Absorptionsvermögen (g/m²) | Optisches Absorptionsvermögen[b] |
|---|---|---|---|---|---|---|
| 1 | 100% PES | 100 g/m² | mechanisch | 3,9 | 390 | 4 |
| 2 | 100% PES | 130 g/m² | mechanisch | 4,1 | 533 | 2 |
| 3 | 100% PES | 150 g/m² | mechanisch | 2,9 | 435 | 2 |
| 4 | 100% PES | 120 g/m² | Thermisch | 1,5 | 180 | 5 |
| 5 | 100% PES | 160 g/m² | thermisch | 1,4 | 224 | 6 |
| 6 | 100% PES | 100 g/m² | mechanisch + thermisch | 3,7 | 370 | 1 |
| 7 | 100% PES | 150 g/m² | mechanisch + thermisch | 3,6 | 540 | 1 |
| 8 | 80% PES / 20% CV | 100 g/m² | chemisch | 2,6 | 260 | 4 |
| 9 | 70% PES / 30% CV | 100 g/m² | mechanisch + thermisch | 3,5 | 350 | 1 |
| 10 | 70% PES / 30% CV | 125 g/m² | mechanisch + thermisch | 3,1 | 388 | 1 |
| 11 | 70% PES / 30% CV | 150 g/m² | mechanisch + thermisch | 3,2 | 480 | 1 |
| 12 | PES / CV | 75 g/m² | chemisch | 3,5 | 263 | 2 |
| 13 | PES / CV | 100 g/m² | thermisch | 1,3 | 130 | 3 |
| 14 | PES / CV | 100 g/m² | thermisch + chemisch | 3,4 | 340 | 4 |
| 15 | PES / CV | 130 g/m² | thermisch + chemisch | 2,9 | 377 | 2 |

[a] Absorptionsvermögen bestimmt über das Ausgangsgewicht des Vliesstoffes und anschließender Rückwiegung nach Eintauchen in VE-Wasser und Abpressen an der Imprägniermaschine, [b] Skala von 1-6 (1 = sehr gut, 6 = ungenügend) [c] Vlies ist nicht stabil (dunkelgrüne Grundfarbe nach einigen Wochen)

| Eintrag | Bezeichnung | $M_w$(PVP) | Ergebnis |
|---|---|---|---|
| 1[*] | Ohne PVP | - | erst ab 5 mg/L $PO_4^{3-}$ |
| 2[*] | N-Methyl-2-pyrrolidon | - | k. R.[a] |
| 3[*] | 1-Dodecyl-2-pyrrolidon | - | k. R. |
| 4[*] | PVPP[b] | - | unlöslich (in Wasser) |
| 5[*] | PVP K25 | 24.000 g/mol | schwache Reaktion |
| 6 | PVP K40 | 40.000 g/mol | gute Farbabstufung |
| 7[*] | PVP K90 | 360.000 g/mol | Falsch positives Ergebnis[c] (schlechte Löslichkeit) |
| 8[*] | PVP K40 / PVP K90 | - | schwache Reaktion |
| 9[*] | PVA[d]- Co-PVP 7:3 | 50.000 g/mol | schwache Reaktion |
| 10[*] | PVA-Co-PVP 3:7 | 50.000 g/mol | schwache Reaktion |
| 11[*] | Luviskol®[e] | - | k. R. |

[a]keine Reaktion, [b]Poly(vinylpolypyrrolidon) - vernetzt - 110 µm Partikelgröße, [c]Der Nullwert zeigt bereits eine grün-blaue Farbe, [d]Polyvinylacetate, [e]Polyvinylcaprolactam

* = nicht gemäß der Erfindung

Figur 4

# Figur 5

| Eintrag | Säure | pH-Wert der Imprägnierlösung | Ergebnis |
|---|---|---|---|
| 1 | Oxalsäure | 1,0 | k. R. |
| 2 | Weinsäure | - | k. R. |
| 3 | Cyclaminsäure | 1,48 | gute Reaktionsfarbe (ab 0,5 mg/L) |
| 4 | Methylamidosulfonsäure[a] | 1,0 | Unterscheidbarkeit ab 3 mg/L |
| 5 | Amidoschwefelsäure | 1,6 | Unterscheidbarkeit ab 1 mg/L |
| 6 | MOPS[b] (+ HCl) | 1,5 | k. R.[c] |
| 7 | MES[d] (+ HCl) | 1,5 | k. R.[c] |
| 8 | Polystyrolsulfonsäure (+ HCl) | 1,0 | k. R.[c] |
| 9 | Polyacrylsäure + Cyclaminsäure (+ HCl) | 0,75 | Reaktionsfarbe ab 3 mg/L |
| 10 | p-Toluolsulfonsäure +Cyclaminsäure | - | Reaktionsfarbe ab 1 mg/L |
| 11 | Polystyrolsulfonsäure+Cyclaminsäure (+ HCl) | 1,0 | fällt aus |
| 12 | Ascorbinsäure + Cyclaminsäure | | Reaktionsfarbe ab 3 mg/L |
| 13 | Cyanursäure + Cyclaminsäure | - | fällt aus |

[a] schlecht löslich, [b] 3-(N-Morpholino)propansulfonsäure, [c] sehr schwache Reaktion, [d] 2-(N-Morpholino)ethansulfonsäure

# Figur 6

| Eintrag | Reduktionsmittel | Ergebnis / Farbe | Bemerkung |
|---|---|---|---|
| 1* | Leuko Bindschedlers Grün | grün-blau | - |
| 2* | Leuko Malachitgrün + TMB[a] | grün | ab 3 mg/L |
| 3* | Leuko Kristallviolett + TMB | grün | ab 3 mg/L |
| 4* | Ascorbinsäure | k. R | - |
| 5* | Syringaldazin + Vanillinazin | k. R | - |
| 6* | Aminophenol | k. R | - |
| 7* | Zink | k. R | - |
| 8 | Leuko Bindschedlers Grün + TMB | grün-blau | ab 0,5 mg/L |
| 9* | Leuko Bindschedlers Grün + Ascorbinsäure | blau | ab 3 mg/L |
| 10* | LBG[b] + Benzoyl Leukomethylenblau | grün-blau | ab 1 mg/L |
| 11* | LBG + 1,2-Phenylendiamin | grün-blau | ab 3 mg/L |
| 12* | LBG + 1,3-Phenylendiamin | grün-blau | ab 1 mg/L |
| 13* | LBG + 1,4-Phenylendiamin | grün-blau | ab 3 mg/L |
| 14* | LBG + o-Tolidin | grün-blau | ab 3 mg/L |

[a]3,3',5,5'-Tetramethylbenzidin, [b]Leuko Bindschedlers Grün

* = nicht gemäß der Erfindung

# Figur 7

| Eintrag | Reduktionsmittel | Konzentration (mg/L $PO_4^{3-}$) | | | | | |
|---|---|---|---|---|---|---|---|
| | | 0,0 | 0,25 | 0,5 | 1,0 | 3,0 | 5,0 |
| 1 * | LBG[a] | Grün-weiß | Grün-weiß | Helles Grün | Grün-blau | Kräftiges Grün-blau | Kräftiges Grün-blau |
| 2 * | TMB[b] | Grün-weiß | Grün-weiß | Helles Grün | Grün-blau | Kräftiges Grün-blau | Kräftiges Grün-blau |
| 3 | *LBG / TMB* | *Weiß* | *Sehr helles Grün* | *Helles Grün* | *Grün-blau* | *Kräftiges Grün-blau* | *Dunkles Grün-blau* |
| 4 | LBG / LMG[c] | Grün-weiß | Sehr helles Grün | Helles Grün | Grün-blau | Kräftiges Grün-blau | Kräftiges Grün-blau |
| 5 | LBG / LKV[d] | Grün-weiß | Sehr helles Grün | Helles Grün | Grün-blau | Kräftiges Grün-blau | Kräftiges Grün-blau |
| 6 * | LBG / 1,3-Phenylendiamin | Weiß-grau | Weiß-grau | Sehr helles Grün | Helles Grün | Grün-blau | Kräftiges Grün-blau |
| 7 | *LBG / MDA[e]* | *Weiß-grau* | *Weiß-grau bis sehr helles Grün* | *Sehr helles Grün* | *Helles Grün* | *Grün-blau* | *Kräftiges Grün-blau* |
| 8 * | LMG / TMB | Weiß-grau | Weiß-grau | Sehr helles Grün | Helles Grün | Grün-blau | Grün-blau |
| 9 * | LKV / TMB | Weiß-grau | Weiß-grau | Sehr helles Grün | Helles Grün | Grün-blau | Grün-blau |

[a] Leuko Bindschedlers Grün, [b] 3,3′,5,5′-Tetramethylbenzidin, [c] Leuko Malachitgrün, [d] Leuko Kristallviolett, [e] 4,4′-Methylen-N,N-bis-dimethylanilin

* = nicht gemäß der Erfindung

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- US 5858797 A
- WO 2017044806 A
- DE 102016202428

### In der Beschreibung aufgeführte Nicht-Patentliteratur

- **MURPHY, J. ; RILEY, J. P.** A modified single solution method for the determination of phosphate in natural waters. *Analytica chim. Acta,* 1962, vol. 27, 31-36

- A kinetic study on the influence of the parameters in the determination of inorganic phosphate by the molybdenum blue reaction. **BARTELS P.C. et al.** CLINICA CHIMICA ACTA. ELSEVIER BV, 02. Juni 1975, vol. 61, 135-144